# EUROPEAN PATENT APPLICATION

(11) **EP 3 666 331 A1**
(43) Date of publication of application: **17.06.2020**
(21) Application number: 18211776.2
(22) Date of filing: 11.12.2018
(51) Int. Cl.: A61N 5/06, A61K 41/00, A61L 2/00

(54) **COMPOSITION FOR THE USE IN TOPICAL ANTIMICROBIAL OR ANTI-INFECTIVE TREATMENT OF SKIN, SOFT TISSUE AND WOUNDS**

(71) Applicant: bredent medical GmbH & Co. KG, 89250 Senden (DE)
(72) Inventor: Vander Beken, Seppe, 89134 Blaustein (DE); Micko, Gerald, 88471 Laupheim (DE)
(74) Representative: Baur & Weber Patentanwälte PartG mbB

(57) **Abstract**

A composition for the use in topical antimicrobial or anti-infective treatment of skin and soft tissue infections is described, the composition includes an antiseptic substance and a photosensitizer at the time of application, wherein the antiseptic substance exerts an antimicrobial or anti-infective effect on microorganisms or their biofilm on skin, soft tissue and wounds and the photosensitizer catalyzes a photodynamic antimicrobial or anti-infective effect upon irradiation with light of appropriate wavelength, power-density and energy-dose, directed at the microorganisms or their biofilm on skin, soft tissue and wounds, for simultaneous application of both the antiseptic substance and the photosensitizer.

## Description

The invention relates to a composition for the use in topical antimicrobial or anti-infective treatment of skin, soft tissue and wounds.

### Background of the Invention

Devices for photodynamic therapy and respective substances for usage with such devices are known.

For example, in US 2012/0296260 A1 an apparatus for photodynamic therapy and/or for destroying or reducing microorganisms is shown comprising an irradiation unit having at least one light source, by means of which a photosensitizer applied to a wound area to be treated is activated by way of irradiation, further comprising a camera for recording images of the wound, which is disposed in the irradiation unit, and a positioning unit, by means of which the irradiation unit can be oriented with respect to the wound area. On a display for the images is a grid with visual indicia distinguishing fields to be irradiated by means of the light sources.

EP 1 737 492 A1 shows a preparation for the photodynamic control of micro-organisms, said preparation being in a liquid or pasty form and containing a photosensitizer which comprises a dyestuff and produces singlet oxygen when irradiated by means of light. The micro-organisms can be marked by means of the dyestuff. The aim is to improve said preparation in order to enable an improved photodynamic control. To this end, the preparation contains an active ingredient for amplifying or weakening the oxidative action of the singulet oxygen by chemical manipulation of the dyestuff or the nano environment thereof. In a particular form, a rinsing solution is used before the irradiation.

More generally other forms of substances for wound treatment are known.

In DE 100 12 026 A1 a gel is described which comprises an aqueous solution containing (by weight) 0.01-0.3% polyhexamethylene biguanide (PHMB), 1-15 wt. % glycerol and 0.2-5% hydroxyethyl cellulose (HEC).

In DE 10 2004 037 598 A1 a medium for use in mouth and throat area is described, which comprises microbicide aqueous solution comprising a linear biguanide polymer and/or water-soluble salt of microbicide in combination with at least a sweetener.

WO 2003/004013 A1 shows a wound treatment agent that contains, in aqueous solution, polyhexamethylene biguanide and at least one surfactant. The surfactant is a glycine derivative and/or a sulfosuccinate and/or an amide based on an unbranched fatty acid. The surfactant is preferably a betaine and, in particular, an amidoalkyl betaine of a fatty acid. The wound treatment agent is excellently suited for use as a washing or shower gel, as a moisturizing gel or as a moist wound covering, as a dissolving gel for dissolving incrustations or scabs from body surfaces or wounds or for removing dressings and for changing moist dressings.

In EP 1 263 465 B1 a method of photoeradication of cellular and acellular organisms is shown which includes the steps of providing a surface acting agent in association with a cellular or acellular organism, the surface acting agent disorienting a membrane structure so that said membrane no longer functions as an effective osmotic barrier; providing a photosensitive material in association with the cellular or acellular organism; and applying light in association with the cellular or acellular organism to cause a disruption of the organism.

WO 2010/070292 A1 shows a composition for use on skin and wounds, comprising a photo-catalyst which is capable of preferentially staining biofilms the composition being for use in the diagnosis and treatment of biofilms in wounds.

### Summary of the Invention

It is therefore an object of the invention to provide a composition which enhances the therapeutic effect during antimicrobial photodynamic therapy.

This object is addressed by independent claim 1. Further advantageous embodiments of the invention are described in the sub-claims. These can be combined in technologically meaningful ways. The specification additionally characterizes and specifies the invention.

According to an embodiment of the invention, a composition for the use in topical antimicrobial or anti-infective treatment of skin, soft tissue and wounds is provided, which comprises an antiseptic substance and a photosensitizer at the time of application, wherein the antiseptic substance exerts an antimicrobial or anti-infective effect on microorganisms or their biofilm on skin, soft tissue and wounds and the photosensitizer catalyzes a photodynamic antimicrobial or anti-infective effect upon irradiation with light of appropriate wavelength, power-density and energy-dose, directed at the microorganisms or their biofilm on skin, soft tissue and wounds, for simultaneous application of both the antiseptic substance and the photosensitizer.

Accordingly, the simultaneous application of both the antiseptic substance and the photosensitizer and the combined corresponding skin, soft tissue and wounds antimicrobial therapy-modalities is therapeutically advantageous over application of the antiseptic substance and the photosensitizer with subsequent photodynamic therapy alone.

The advantage is achieved for one or more of the following reasons by the combined therapy. The combined application of the antiseptic substance and the photosensitizer with subsequent antimicrobial photodynamic therapy results in a broader antimicrobial and or anti-infective spectrum. An additive or synergistic antimicrobial or anti-infective effect on microorganisms and their biofilm possibly causing the skin and soft tissue infections can be achieved by the combined application of the antiseptic substance and the photosensitizer-mediated antimicrobial photodynamic therapy, which also reduces the risk for tolerance- or resistance-development by the targeted microorganisms. A reduction of the effective concentration of both substances can be achieved in the inventive composition, which lowers the risk of unwanted side effects by the respective substances on the patient.

According to further embodiments of the invention, the antiseptic substance is polyhexamethylene biguanide (PHMB) and the photosensitizer is a phenothiazinium-class molecule, preferably methylene blue (MB).

Using PHMB and MB for the two substances of the inventive composition is preferred and achieves the therapeutically advantageous results. More generally, the class of phenothiazinium photosensitizers is well studied, documented and approved of in topical applications for antimicrobial photodynamic therapy and comprises the dyes MB and its derivatives, including new methylene blue and 1,9-dimethyl-methylene blue, as well toluidine blue O (TBO) and its derivatives.

Antiseptic substances are generally used and well established in clinical practice. Presently these substances are standard of care as a stand-alone product for the prevention and treatment of critical colonization and infection of skin, soft tissue and wounds with pathogenic microorganisms. These substances include the mentioned PHMB, while other known antiseptic substances, indicated for antimicrobial or anti-infective treatment of skin, soft tissue and wounds, could be used as well.

According to further embodiments of the invention, both the antiseptic substance and the photosensitizer each are present in an amount, sufficient to have or mediate an antimicrobial or anti-infective effect. Both the antiseptic substance and the photosensitizer each are advantageously present in an amount below their respective toxicity levels for host animal/human cells and tissue, which provides for therapeutically advantageous results without side effects or damages.

According to further embodiments of the invention, the composition can be provided as a hydro-gel or an aqua-gel. In this respect, the composition can be further comprising a polymeric gelling substance and water.

This is in particular advantageous, when the composition is directly applied during treatment, for example in autolytic wound debridement and/or photodynamic therapy. In other embodiments, the composition according to the invention can also be embedded into a medical dressing or a patch.

The composition can further comprise substances for modifying the physico- and physiochemical properties of the formulation, including pH, tonicity, stability, preservation, viscosity, surface-activity and emulsification as well as skin permeation enhancers, humectants and antioxidants and/or substances for neutralizing bad odors caused by skin, soft tissue and wound infections. Adding such further substances enhances the application of the composition.

The use of the inventive composition in topical antimicrobial or anti-infective treatment can be extended to the application to body orifices and associated mucosae. Advantageously, the oral cavity including tongue, upper throat, tonsils, gum, gingiva, teeth and implants, as well as the nose, the auditory canal can be treated in addition or instead of bodily external skin areas.

In this case the composition may further comprise a substance for modifying taste or sweetness of the composition.

Furthermore, the composition may be used with respect to anorectal and genital mucosae.

It is particularly advantageous using the above-described composition in photodynamic therapy utilizing at least one wavelength being selected in the range of the absorption maximum of the photosensitizer and utilizing power density and energy dose ranges to allow for an antimicrobial or anti-infective effect mediated by the photosensitizer without causing damage to the skin, soft tissue or wound.

According to another embodiment of the invention, the use of the inventive composition can be extended to non-topical antimicrobial or anti-infective treatment applications including by ingestion, by injections or by instillation in surgically created openings to the body.

According to yet another embodiment of the invention, the use of the inventive composition can be extended to industrial antimicrobial or anti-infective applications including food preservation, decontamination of foodstuff, decontamination of aquaculture-, waste- and drinking-water as well as disinfection of surfaces and devices.

### Detailed Description of the Invention

In the following, embodiments of the invention will be explained in more detail.

The invention refers to a composition for the use in topical antimicrobial or anti-infective treatment of skin, soft tissue and wounds.

The composition comprises an antiseptic substance and a photosensitizer simultaneously at the time of application.

In general, skin, soft tissue and wound infections refer to the critical colonization or infection with potentially pathogenic microorganisms and their biofilm of the skin, skin wound or ulcer and underlying soft tissues, leading to infectious disease and symptoms including tissue inflammation and pain, stalled wound healing or even tissue damage and necrosis.

An antimicrobial effect, i.e. the killing of potentially pathogenic microorganisms or destruction of its biofilm, and anti-infective effect or the prevention of serious local spreading and systemic infection with such microorganisms, is advantageous for the patient for reasons that include enhanced wound healing or infective disease prevention and resolution.

A broad antimicrobial spectrum and efficiency and at the same time a low toxicity to the treated skin, soft tissue or wound are advantageous characteristics of a composition for antimicrobial or anti-infective treatment.

The antiseptic substance exerts an antimicrobial or anti-infective effect on microorganisms or their biofilm causing skin and soft tissue infections. The antiseptic substance can be polyhexamethylene biguanide (PHMB), but alcohols (ethanol, isopropanol, etc.), quaternary ammonium compounds (benzalkonium chloride, benzethonium chloride, methylbenzethonium chloride, cetalkonium chloride, cetylpyridinium chloride, cetrimonium, cetrimide, dofanium chloride, tetraethylammonium bromide, didecyldimethylammonium chloride and domiphen bromide), chlorhexidine gluconate, octenidine dihydrochloride, hydrogen peroxide,sodium hypochlorite, hypochlorous acid or antiseptic solutions like electrochemically activated water (ECA) or liquid plasma could be used as well. Advantageously, the antiseptic substance absorbs as less light as possible at the wavelengths used for simultaneous photodynamic therapy with the inventive composition or does not chemically abrogate the photo-catalyzing properties of the photosensitizer when combined within the inventive composition.

The photosensitizer catalyzes a photodynamic antimicrobial or anti-infective effect upon irradiation with light of appropriate wavelength, power-density and energy-dose, directed at the microorganisms or their biofilm possibly causing skin, soft tissue and wound infections.

Typically, phenothiazinium-class photosensitizers are the well-studied antimicrobial dye-type photosensitizers for topical use that could be advantageously used in combination with an antiseptic substance. However, other such synthetic dye antimicrobial photosensitizers (e.g. rose bengal, erythrosine, indocyanine green, crystal violet) as well as other classes of antimicrobial photosensitizers, such as cyclic polypyrroles (e.g. porphyrins, chlorins, bacteriochlorins, phthalocyanines) and other natural (e.g. psoralens, perylenequinones, curcumin, riboflavin) or synthetic (e.g. boron-dipyrromethene and squaraine-based chromophores, fullerenes) photoactive compounds could be used as well. Phenothiazinium-class photosensitizers comprise the preferred dye methylene blue (MB) and its derivatives, including new methylene blue and 1,9-dimethyl-methylene blue, as well toluidine blue O and its derivatives.

The combined application of both the antiseptic substance and the photosensitizer for simultaneously performing both corresponding disinfecting therapy-modalities for skin, soft tissue and wound infections are therapeutically advantageous over each single modality alone.

First, a broader antimicrobial and or anti-infective spectrum can be achieved. Each single antimicrobial or anti-infective therapeutic modality is associated with a specific antimicrobial spectrum that may largely overlap but also shows differences in efficacy against certain types of microorganisms. The simultaneous application of both modalities therefore has an extended antimicrobial spectrum as compared to each one of the single modalities applied alone.

Second, an additive or synergistic antimicrobial or anti-infective effect on microorganisms and their biofilm on the skin, soft tissue and wounds can be achieved. Both substances can sensitize microorganisms for the other antimicrobial or anti-infective modality, respectively, by improving bioavailability and/or localization of the other substance to said microorganisms and their biofilm. This causes an improved effectiveness by the combined-modalities application and reduces the risk for tolerance- or resistance-development by microorganisms causing the skin and soft tissue infections to each said single therapy-substance. As an example, this is especially important for gram-negative pathogenic bacteria, such as Pseudomonas aeruginosa, a bacterial species often associated with biofilms of found in hard to heal wounds. Pseudomonas aeruginosa infections are difficult to treat with either PHMB or MB-mediated photodynamic therapy alone but are less tolerant to the combined antimicrobial treatment approach.

Third, a reduction of the effective concentration of both substances needed in the composition can be achieved, which lowers the risk of unwanted side effects by the respective substances on the patient. Overall, the benefit-over-risk ratio improves, meaning that the therapeutic efficiency increases whilst unwanted side effects can be reduced or prevented.

Typically, the composition is provided as a hydro-gel or an aqua-gel and further comprises a polymeric gelling substance and water. Accordingly, the composition can be directly applied during treatment, for example in autolytic wound debridement and/or photodynamic therapy. It is however also possible embedding the composition into a medical dressing or a patch.
Typically, polymeric gelling substances for topical applications are natural proteins (e.g. gelatin, casein, collagen, egg whites, polysaccharides), semisynthetic cellulose subordinates (e.g. carboxymethyl cellulose, ethylcellulose, hydroxyethylcellulose, hydroxypropyl cellulose, hydroxypropylmethyl cellulose, Mg/AI-silicate, methylcellulose, sodium alginate) or fully synthetic carbomers, poloxamers, polyvinyl alcohols and others.

The composition can further comprise substances for modifying the physico- and physiochemical properties of the formulation, including pH, tonicity, stability, preservation, viscosity, surface-activity and emulsification as well as skin permeation enhancers, humectants and antioxidants and/or substances for neutralizing bad odors caused by skin and soft tissue infections. Adding such further substances enhances the application of the composition.

The use of the composition in topical antimicrobial or anti-infective treatment can be extended to the application to body orifices and associated mucosae. Advantageously, the oral cavity including tongue, upper throat, tonsils, gum, gingiva, teeth and implants, as well as the nose, the auditory canal can be treated in addition or instead of skin areas. In this case the composition may further comprise a substance for modifying taste or sweetness of the composition. Furthermore, the composition may be used with respect to anorectal and genital mucosae.

According to another embodiment of the invention, the use of the inventive composition can be extended to non-topical antimicrobial or anti-infective treatment applications including by ingestion, by injections or by instillation in surgically created openings to the body. In particular, instillation into a surgically created opening can be advantageous for antimicrobial treatment or disinfection of cysts or implant infections. Hereto, the composition as well as the optical device or probe for light application for applying the photodynamic therapy and that enter the body need to be sterile.

According to yet another embodiment of the invention, the use of the inventive composition can be extended to industrial antimicrobial or anti-infective applications including food preservation, decontamination of foodstuff, decontamination of aquaculture-, waste- and drinking-water as well as disinfection of surfaces and devices. These represent novel domains in which the application of antimicrobial photodynamic therapy is potentially advantageous for disinfection purposes.

The combined application of a composition comprising both a photosensitizer for photodynamic treatment and an antiseptic substance at the time of application for said extended medical and industrial uses can be advantageous for the same reasons as for the antimicrobial or anti-infective topical application to skin, soft tissue or wounds.

In the following, experimental results are provided. In vitro experiments to kill Pseudomonas aeruginosa germs in a wound-representative semi-solid biofilm model have been performed. In this respect setup A refers to no treatment at all. Setup B and C use either PHMB as the antiseptic substance or MB as the photosensitizer. Setup D refers to the invention, where both PHMB as the antiseptic substance and MB as the photosensitizer are used.

For setup C and D, i.e. those utilizing a photosensitizer, the respective light irradiation have been performed with the same values for wavelength, power-density and energy-dose. For all experimental setups, the fractions of gelling substance and water have been adapted so as to arrive at the same viscosity. The survival rate of the microorganisms is expressed in the usual terms of colony-forming units (CFU). The results are summarized in the following table:

| Setup | % PHMB | % MB | Mean CFU/well (± SD), relative to A |
|---|---|---|---|
| A | 0 | 0 | 100% (± 24,1%) |
| B | 1,0 | 0 | 19,7% (± 9,7%) |
| C | 0 | 0,02 | 28,3% (± 6,2%) |
| D | 1,0 | 0,02 | 1,8% (± 1,6%) |

The inventive composition can be used together with a therapy device for irradiating a surface of a tissue, in particular skin tissue.

The above and the features indicated in the claims can be realized advantageously both individually and in various combinations. The invention is not limited to the embodiments described herein, but can be modified in many ways within the knowledge of a person skilled in the art.

## Claims

1. A composition for the use in topical antimicrobial or anti-infective treatment of skin, soft tissue and wounds, said composition comprising an antiseptic substance and a photosensitizer at the time of application, wherein the antiseptic substance exerts an antimicrobial or anti-infective effect on microorganisms or their biofilm on skin, soft tissue and wounds and the photosensitizer catalyzes a photodynamic antimicrobial or anti-infective effect upon irradiation with light of appropriate wavelength, power-density and energy-dose, directed at the microorganisms or their biofilm on skin, soft tissue and wounds, for simultaneous application of both the antiseptic substance and the photosensitizer.

2. The composition according to claim 1, wherein the antiseptic substance is polyhexamethylene biguanide (PHMB).

3. The composition according to claim 1 or 2, wherein the photosensitizer is phenothiazinium-class molecule, preferably methylene blue (MB).

4. The composition according to any of claims 1 to 3, wherein both the antiseptic substance and the photosensitizer each are present in an amount, sufficient to have an antimicrobial or anti-infective effect.

5. The composition according to claim 4, wherein both the antiseptic substance and the photosensitizer each are present in an amount below their respective toxicity levels for host animal/human cells and tissue.

6. The composition according to any of claims 1 to 5, which is provided as a hydro-gel or an aqua-gel comprising a polymeric gelling substance and water.

7. The composition according to any of claims 1 to 6, which is provided within a medical dressing device or a patch.

8. The composition according to any of claims 1 to 7, which further comprises substances for modifying the physico- and physiochemical properties of the formulation, including pH, tonicity, stability, preservation, viscosity, surface-activity and emulsification as well as skin permeation enhancers, humectants and antioxidants, and/or substances for neutralizing bad odors caused by skin and soft tissue infections.

9. The composition according to any of claims 1 to 8, which is used in topical antimicrobial or anti-infective treatment being extended to the application to body orifices and associated mucosae.

10. The composition according to claim 9, further comprising a substance for modifying taste or sweetness of the composition.

11. Use of a composition according to any of claims 1 to 10 in photodynamic therapy utilizing at least one wavelength being selected in the range of the absorption maximum of the photosensitizer.

12. Use of a composition according to any of claims 1 to 10 in non-topical antimicrobial or anti-infective treatment applications including by ingestion, by injections or by instillation in surgically created openings to the body.

13. Use of a composition according to any of claims 1 to 10 in industrial antimicrobial or anti-infective applications including food preservation, decontamination of foodstuff, decontamination of aquaculture-, waste- and drinking-water as well as disinfection of surfaces and devices.
